(19) Europäisches Patentamt
European Patent Office
Office européen des brevets

(11) EP 1 014 954 B1

(12) **EUROPÄISCHE PATENTSCHRIFT**

(45) Veröffentlichungstag und Bekanntmachung des Hinweises auf die Patenterteilung:
**02.07.2003 Patentblatt 2003/27**

(51) Int Cl.[7]: **A61K 9/70**, A61K 9/00, A61K 9/06, A61K 47/00, A61K 47/22

(21) Anmeldenummer: **98907826.6**

(22) Anmeldetag: **13.01.1998**

(86) Internationale Anmeldenummer:
**PCT/DE98/00157**

(87) Internationale Veröffentlichungsnummer:
**WO 98/030203 (16.07.1998 Gazette 1998/28)**

(54) **TRANSDERMALES THERAPEUTISCHES SYSTEM**
TRANSDERMAL THERAPEUTIC SYSTEM
SYSTEME THERAPEUTIQUE TRANSDERMIQUE

(84) Benannte Vertragsstaaten:
**AT BE CH DE DK ES FI FR GB GR IE IT LI LU NL PT SE**

(30) Priorität: **13.01.1997 DE 19701949**

(43) Veröffentlichungstag der Anmeldung:
**05.07.2000 Patentblatt 2000/27**

(73) Patentinhaber:
- **Jenapharm GmbH & Co. KG
07745 Jena (DE)**
- **LTS Lohmann Therapie-Systeme AG
56626 Andernach (DE)**

(72) Erfinder:
- **DITTGEN, Michael
D-99510 Apolda (DE)**
- **FRICKE, Sabine
D-07749 Jena (DE)**
- **VÖLKEL, Christoph
D-07743 Jena (DE)**
- **AHRENS, Kathrin
D-64354 Reinheim (DE)**
- **GERECKE, Hagen
D-07743 Jena (DE)**
- **KÖPKE, Kai
CH-5042 Hirschthal (CH)**

(74) Vertreter: **Flaccus, Rolf-Dieter, Dr. et al
Patentanwalt
Bussardweg 10
50389 Wesseling (DE)**

(56) Entgegenhaltungen:
**EP-A- 0 152 281** **EP-A- 0 410 696**
**EP-A- 0 425 968** **EP-A- 0 622 075**
**EP-A- 0 732 100** **WO-A-93/02669**
**WO-A-93/08795** **WO-A-94/10984**
**WO-A-94/22481** **WO-A-95/19162**
**WO-A-95/22321** **WO-A-95/22322**
**AT-B- 393 624** **DE-C- 4 410 637**
**US-A- 4 777 047** **US-A- 4 879 119**
**US-A- 5 422 361**

- **DATABASE WPI Section Ch, Week 9440 Derwent Publications Ltd., London, GB; Class A96, AN 94-322096 XP002900196 & JP 06 247856 A (SHISEIDO CO LTD) , 6. September 1994**
- **DATABASE WPI Section Ch, Week 8619 Derwent Publications Ltd., London, GB; Class B05, AN 86-123252 XP002900197 & JP 61 063615 A (NAITO S.), 1. April 1986**
- **DATABASE WPI Section Ch, Week 8007 Derwent Publications Ltd., London, GB; Class A96, AN 80-11979c XP002900198 & JP 55 002604 A (SANWA KAGAKU KENKYUSHO CO), 10. Januar 1980**
- **AKHTER, S.A. ET AL.: "Penetration Enhancers in Human Skin - Effect of Oleic Acid and Azone on Flurbiprofen Permeation" JOURNAL OF PHARMACY AND PHARMACOLOGY, BRITISH PHARMACEUTICAL CONFERENCE 1984, SCIENCE PROCEEDINGS, 121ST MEETING, 11-14 SEPTEMBER 1984, SOUTHAMPTON, Bd. 36, Dezember 1984, XP002900200 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein transdermales therapeutisches System zur Anwendung auf der Haut und/oder Schleimhaut bestehend aus mindestens einem Wirkstoffs in Form einer festen Dispersion in Kombination mit mindestens einem Strukturbrecher und/oder mindestens einem Strukturbildner in einer gemeinsamen Matrix.

Die Erfindung betrifft somit ein Verfahren zur Verbesserung der Hautpermeation von Wirkstoffen, das insbesondere zur Herstellung eines transdermalen therapeutischen Systems (TTS) eingesetzt werden kann, das zur Anwendung auf der Haut oder Schleimhaut bestimmt ist.

Der Terminus "TTS" beschreibt eine Applikationsvorrichtung, die auf dem Zielorgan Haut oder Schleimhaut haftet und dabei den enthaltenen Arzneistoff durch das Zielorgan hindurch im Organismus systemisch wirksam werden läßt.

Die Begriffe Strukturbrecher und Strukturbildner entstammen der "Eis"-Therorie der Hydrogele, wie beispielsweise von Hüttenrauch et al. beschrieben (Pharmazie 40, S. 427, 1985).

Zusammensetzungen, die zur transdermalen Verabreichung von Wirkstoffen dienen, sind in vielerlei Formen bekannt:

US-A 4,777,047 beschreibt Formulierungen zur transdermalen Applikation, welche einen Calcium-Antagonisten und oberflächenaktive Hilfsstoffe wie Isopropylmyristat oder Ethyloleat in einem Lösungsmittel enthalten. Als Lösungsmittel werden allgemein Propylenglykol, Linolensäure, Oleylalkobol, Solketal oder Dimethylsulfoxid genannt.

US-A 5,422,361 beschreibt eine Creme oder Lotion, die einen lipophilen pharmazeutischen Wirkstoff enthält. Als Grundmaterial wird dabei eine physikalisch und chemisch stabile Öt-in-Wasser-Emulsion verwendet, die einen Gehalt an N-Methyl-2-Pyrrolidon, Dimethylsulfoxid, Solketal oder Oleylalkohol aufweist. Für Solketal und Dimetnylsulfoxid sind Grundmaterialien beschrieben, die einen Gehalt an diesen Stoffen von höchstens 10 Gew.-% aufweisen.

DE-C 43 09 830 beschreibt ein Wirkstoffpflaster für die Abgabe von Estradiol an die Haut. Das Wirkstoffpflaster weist ein Wirkstoffreservoir aus einem Haftkleber auf. In der Polymermatrix des Haftklebers ist zur Verbesserung der Bioverfügbarkeit des Estradiols ein Penetrationsbeschleuniger, nämlich Monoisopropyliden-Glycerin (MIPG, Solketal) oder Monoisopropyliden-Diglycerin (MIPD) enthalten.

Akhter, S. A. et al. (J. Pharm. Pharmakol. 36, Suppl., S. 7 (1984)) beschreiben Lösungen eines Wirkstoffs im Lösungsmittel Solketal, denen als penetrationsvertärkendes Agens 7 % Ölsäure zugesetzt ist.

US-A 4,879,119 beschreibt ein Pflaster, das einen Behälter aus Polyvinylchlorid zur Applikation von Calciumantagonisten und beta-Blockern umfaßt. Die Wirkstoffe werden bei diesem Pflaster in Fetten oder Ölen dispergiert, vorzugsweise in Kakaobutter, Isokakaobutter oder Triglyceriden, deren Fettsäuren 12 bis 18 C-Atome lang sind. Die wirkstoffhaltige, flüssig bis pastöse Masse wird in den Behälter gefüllt und gegebenenfalls mit einem mit Polyethylenglykol imprägnierten Filterpapier abgedeckt, das ein unbeabsichtigtes Auslaufen der wirkstoffhaltigen Masse verhindern soll. Bei der Anwendung eines Pflasters nach US 4,879,119 schmelzen die Öle oder Fette durch die Körperwärme des Trägers und sollen eine gute Hautpermeation des Wirkstoffes bewirken.

EP 0 152 281 A beschreibt eine transdermale Formulierung zur Verabreichung von Nicardipinhydrochlorid, das in einer Flüssigkeit, die Harnstoff und zumindest eine weitere Verbindung enthält, gelöst oder suspendiert wird. Die weitere Verbindung kann ein Polyalkohol sein, so dass die gemeinsame Verwendung von Harnstoff und einem Polyalkohol die Permeation von Nicardipin verbessern soll.

WO 95/22 321 offenbart ein Desogestrel enthaltendes Mittel zur transdermalen Applikation, in dem auch ein Östrogen oder auch zwei Östrogene miteinander kombiniert vorhanden sind. Ferner enthält dieses Mittel ein oder zwei miteinander mischbare penetrationsverstärkende Agenzien.

In WO 95/ 22 322, eine Parallelanmeldung zu der zuvor aufgeführten WO 95/22 321, werden transdermale therapeutische Systeme beschrieben, die Sexualsteroide und als obligatorischen Bestandteil Dimethylisosorbid enthalten, wobei nicht fließfähige Gelphasen ausgenommen sind.

WO 95/19 162 beschreibt Carbonsäuren als Penetrationsverstärker für Estradiol.

WO 94/22 481 offenbart die Verwendung von Monoisopropylidenglycerol (MIPG) oder Monoisopropylidendiglycerol als penetrationsverstärkende Agenzien für Estradiol. Um die Hautpermeation von Estradiol weiter zu erhöhen, wird die Zugabe weiterer Verbindungen aus anderen chemischen Klassen vorgeschlagen, ohne dass diesbezüglich lipophile Verbindungen erwähnt werden.

Derartige Darreichungsformen zur transdermalen Applikation mögen für gewisse Zwecke, insbesondere für Wirkstoffe, für welche die Haut relativ gut permeabel ist, ausreichend sein. In der Regel ist die Epidermis beispielsweise von Menschen jedoch für Wirkstoffe relativ wenig permeabel. Bei Verwendung der bekannten Formulierungen wird demzufolge meist zu wenig an Wirkstoff über die Haut in die Blutbahn transportiert Außerdem kommt es häufig zu Hautunverträglichkeiten, wie etwa Hautreizung oder gar allergischen Effekten. Dies gilt insbesondere dann, wenn Steroidhormone als Wirkstoffe eingesetzt werden sollen.

Das vorzugsweise Einsatzgebiet transdermaler Arzneiformen sind Beschwerden, Krankheiten, Mangelzustände und ähnliche Notwendigkeiten, wie Übelkeit, Herz-Kreislaufversagen, Hormonmangel, der Wunsch nach Empfängnisverhütung. Diese Notwendigkeiten erfordern eine länger anhaltende, gleichmäßige oder dem biologischen Rhythmus des Blutspiegels angepaßte Bereitstellung des Wirkstoffs.

Typische TTS setzen den enthaltenen Arzneistoff über einen längeren Zeitraum gleichmäßig frei. Darüber hinaus sind jedoch auch kompliziertere Systeme und Mischformen (mixed systems) beschrieben worden. Als Beispiele für TTS werden von D'Mello (Transdermal Patch Drug Delivery, Scrip report BS750, PJB Publications Ltd., 1995) oder in "Rote Liste" (Hrsg.: Bundesverband der pharmazeutischen Industrie (BPI), 1996) erwähnt:

- Nicotine Patches,
- Hyoscine Patch,
- Glyceryl trinitrate patches,
- NSAID patches,
- Fentanyl Patch,
- Clonidine Patch,
- Oestradiol Patch,
- Oestradiol/Norethisterone Patch,
- Estradiol Vaginal rings,
- Isosorbide dinitrate ointments,
- Isosorbide dinitrate transdermal sprays,
- Glyceryl trinitrate ointments.

TTS für Insulin sowie anderer peptidische Wirkstoffe, einschließlich bestimmter "releasing Hormones", befinden sich in Vorbereitung.

Die Herstellung der bisher bekannten TTS berücksichtigt bereits vielfach, daß nicht alle Wirkstoffe in ausreichendem Maße die Haut permeieren. Die einwandfreie Funktion der Systeme hängt jedoch entscheidend von einer gesicherten Permeation ab.

Nach einem aktuellen Überblick (Ghosh, T. K., Banga, A. K., Pharm. Technol., **17** (March) 72-96 (1993) und **17** (April) 62-87 (1993)) gibt es physikalische, chemische und biologische Möglichkeiten, die Hautpermeation zu verbessern. Als chemische Möglichkeit ist die Verwendung sogenannter Penetrationsvermittler zu verstehen. Diese Stoffe dringen in die Haut ein und interagieren mit den Bestandteilen der Hornschicht, dem Haupthindernis für den eindringenden Wirkstoff. Penetrationsvermittler vermindern den Hautwiderstand und steigern so den Hautdurchtritt (Flux) des Wirkstoffes. Zumeist beeinflussen sie zugleich das Verteilungsverhältnis des Wirkstoffs zwischen Haut und Vehikel positiv (Franz, T.J., Tojo, K., Shah, K.R., Kydonieus, A., Transdermal Delivery, in Kydonieus, A. (Ed.) Treatise on Controlled Drug Delivery, Marcel Dekker, Inc., 341-422 (1992); Loth, H., Meth. and Find. Exp. Clin. Pharmacol., **11** (3), 155-164, (1989); Robson, D.L., Thesis, University of Bradford, Postgraduate School of Studies in Pharmacy, 1988, p. 1-25).

Der transdermale Flux erfolgt hauptsächlich interzellulär. Dabei durchdringt der permeierende Stoff die lipophilen Zellstrukturen (lipophile Route), so daß Penetrationsvermittler, die diese Route beeinflussen sollen, je nach Region der Zellstruktur (vgl. Fartasch, M. The nature of the epidermal barrier: Structural aspects. Advan. Drug Delivery Rev. **18**(3),273-282 (1996)) drei Angriffspunkte haben (Barry, B.W., J. Controlled Release, **15,** 237-248, (1991)), nämlich

- in unmittelbarer Nähe polarer Kopfgruppen (Region A)
- in der wäßrigen Region zwischen den Kopfgruppen (Region B) und
- innerhalb der unpolaren Bestandteile der Lipiddoppelschichten (Region C).

Für die meisten Permeationsvermittler ist der Angriffspunkt bekannt, für einige Stoffe fehlen noch genaue Angaben (Tabelle 1).

Tabelle 1

| Einteilung der Penetrationsvermittler nach dem Angriffspunkt | |
|---|---|
| Angriffspunkt (vorwiegend) | Penetrationsvermittler |
| Region A | Wasser, Dioxolan-Derivate, Ethylacetat, Harnstoff*, Ethanol und kurzkettige einwertige Alkohole ($C_2$-$C_6$)*, Propylenglykol* |
| Region B | Ethanol |

beeinflußt zusätzlich: * Keratin-Fibrillen

Tabelle 1 (fortgesetzt)

| Einteilung der Penetrationsvermittler nach dem Angriffspunkt | |
|---|---|
| Angriffspunkt (vorwiegend) | Penetrationsvermittler |
| Region C | DMSO*, DMF**, Laurocapram und Derivate, Fettsäuren (z.B. Ölsäure), Tenside (z. B. Decylmethylsulfoxid), Terpene |
| keine klare Zuordnung | Isopropanol, Glycerin, einwertige Alkohole ($C_8$-$C_{14}$), Alkane, Alkylhalogenide, Amide, Pyrrolidonderivate, Fettsäureester, Cyclodextrine, Polyethylenglycole |

beeinflußt zusätzlich: * Keratin-Fibrillen

** Region A (Solvatation)

Die Übersicht in Tabelle 1 verdeutlicht sehr klar, daß für die bekannten Permeationspromotoren beinahe ausschließlich die Regionen A und C die Angriffspunkte bilden. Diese Promotoren zielen darauf ab, entweder die Kopfgruppen oder die lipophilen Ketten zu beeinflussen. Nur für Ethanol wird der Angriff in der wäßrigen Region in der Umgebung der Kopfgruppen gesehen. Somit kann gesagt werden, daß die bekannten Permeatoren mit Ausnahme von Ethanol insbesondere die lipophilen Regionen beeinflussen und den lipophilen Flux befördern.

Andererseits ist bekannt, daß eine Veränderung der lipophilen Regionen mit schwerwiegenden Beeinträchtigungen des Hautzustandes einhergeht. Diese Beeinträchtigungen reichen von Austrocknung und Versprödung bis hin zu Rissen, deutlichen Irritationen, Rötungen, Ekzemen und ähnlichen Schädigungen der Haut. Dies trifft übrigens auch für Ethanol zu, dessen Konzentration bei dermaler Anwendung daher begrenzt ist. Außerdem hat Ethanol den Nachteil der leichten Verdunstung, so daß Übersättigungszustände auftreten, deren Nachteile später noch erläutert werden.

Es ist jedoch bekannt, daß der Flux auf zwei Wegen möglich ist, auf der schon erwähnten "nonpolar route" und außerdem auf dem Wege einer Porendiffunsion ("polar route") (Yamashita, F., Bando, H., Koyama, Y., Kitagawa, S., Takakura, Y., Hashida, M., In Vivo and in Vitro Analysis of Skin Penetration Enhancement Based on a Two-Layer Diffusion Model with Polar and Nonpolar Routes in the Stratum Corneum, Pharm. Res. **11**, 185-191 (1994).

Beide Fluxe addieren sich gemäß Gleichung 1:

$$J = J_L + J_P \qquad \text{(Gl.1)}$$

mit J Gesamtflux, $J_L$ lipophiler Flux, $J_P$ Porenflux

Im allgemeinen dominiert der lipophile Flux. Daher ist die Haut insbesondere für hydrophile Arzneistoffe kaum durchlässig, zumal der Flächenanteil der für den Porenflux zur Verfügung stehenden Poren und auch die Größe der Poren (0,38 - 1,58 nm) vergleichsweise gering sind.

Zur Verbesserung des Porenflux wurde bereits vorgeschlagen (Hatanaka, T., Manabe, E., Sugibayashi, K., Morimoto, Y., An Application of the Hydrodynamic Pore Theory to Percutaneous Absorption of Drugs, Pharm. Res. **11**, 654-658 (1994)), ein Lösungsmittel mit einer hohen Hautpermeabilität zu verwenden. Die Autoren postulierten, daß auf diese Weise in dem Lösungsmittel gelöste Wirkstoffe durch die Haut geschleust werden können. Am Beispiel ANP und Isosorbiddinitrat (ISDN) als Wirkstoff stellte sich heraus, daß das Verfahren bei diesem vorwiegend auf dem lipophilen Wege diffundierenden Arzneistoff versagt.

Die bisher bekannten Verfahren zur Herstellung einer transdermalen Arzneiform weisen somit überwiegend den erheblichen Nachteil auf, daß bestimmte, in gewissem Maße hydrophile Wirkstoffe, beispielsweise bestimmte Hormone nur in ungenügendem Maße durch die Haut gebracht werden können. Ausdruck dieser Tatsache ist beispielsweise ein Testosteronpflaster, das scrotal angewendet werden muß, wenn das Hormon in ausreichender Menge transdermal resorbiert werden soll. Das vorgeschlagene Verfahren zur Verbesserung des Porenfluxes mit geeigneten Lösungsmitteln in der von Hatanaka, T., Manabe, E., Sugibayashi, K., Morimoto, Y. publizierten Variante erscheint erfolgversprechend, jedoch werden nur relativ geringe Fluxe erreicht, und der Beweis steht noch aus, ob tatsächlich Wirkstoffe dem Lösungsmittelflux folgen. Im Falle ISDN hatte das Verfahren versagt, weil die Substanz allein bereits um den Faktor 10 besser durch die Haut permeiert als das zur Beschleunigung eingesetzte ANP.

US-A-4,379,454 beschreibt eine Abhängigkeit des Wirkstofffluxes (Estradiol) vom Enhancerflux (Ethanol). Es wurde dabei gefunden, daß eine erhöhte Permeationsrate von Ethanol die Permeation von Estradiol ebenfalls erhöht. Allerdings besteht ein Nachteil in dem Potential von Ethanol, die Hautschichten bei längerer Anwendung zu irritieren.

Aufgabe der vorliegenden Erfindung ist es daher, ein TTS zur Verfügung zu stellen, das die vorgenannten Nachteile des Standes der Technik überwindet.

Aufgabe der Erfindung ist es deshalb, Zusammensetzungen zur transdermalen Verabreichung bereitzustellen, die gegenüber den bekannten Zusammensetzungen, insbesondere in bezug auf die Penetrationsfähigkeit bestimmter Wirkstoffe, wie etwa steroidaler Agenzien oder Sexualhormone, verbessert sind. Desweiteren sollen möglichst die bei Verwendung der bekannten Zubereitungen, sei es in Form eines transdermalen therapeutischen Systems (TTS), einer Creme, oder einer Lotion, auftretenden Nachteile, insbesondere Hautreizungen, vermieden werden.

Die Aufgabe wird erfindungsgemäß dadurch gelöst, daß ein transdermales therapeutisches System zur Anwendung auf der Haut und/oder Schleimhaut geschaffen wird, bestehend aus mindestens einem Wirkstoffs in Form einer festen Dispersion in Kombination mit mindestens einem Strukturbrecher, bei dessen alleiniger Verwendung die Relaxationszeit des transdermalen therapeutischen Systems mehr als 120 ms betragen würde, und/oder mindestens einem Strukturbildner, bei dessen alleiniger Verwendung die Relaxationszeit des transdermalen therapeutischen Systems weniger als 119 ms betragen würde, in einer gemeinsamen Matrix.

Bevorzugterweise ist das erfindungsgemäße transdermales therapeutisches System, dadurch gekennzeichnet, daß die feste Dispersion eine molekulardisperse Verteilung des Wirkstoffs in einer inerten Trägersubstanz ist.

Erfindungsgemäß ist die inerte Trägersubstanz aus an sich bekannten inerten Trägerstoffen für feste Dispersionen, wie beispielsweise Saccharose, Lactose, Bernsteinsäure Polyethylenglycolen, Polyvinylpyrrolidon, Harnstoff, Mannitol, Mannitose oder deren Mischungen ausgewählt.

Erfindungsgemäß ist der Strukturbrecher aus der Gruppe der Carbonsäureamide, wie beispielsweise Harnstoff, Nicotinsäureamid, Bernsteinsäureamid, Methylacetamid, Ethylacetamid oder deren Mischungen ausgewählt.

Bevorzugterweise beträgt bei dem erfindungsgemäßen transdermales therapeutisches System die Relaxationszeit mehr als 120 ms, vorzugsweise mehr als 150 ms.

Erfindungsgemäß ist der Strukturbildner aus der Gruppe der Polyole, wie beispielsweise Glycerin, Ethylenglycol, Propylenglycol, aus der Gruppe der Zuckeralkohole, wie beispielsweise Sorbitol und/oder aus der Gruppe der Zucker, wie beispielsweise Saccharose oder Glucose oder deren Mischungen ausgewählt.

Bevorzugterweise beträgt hierbei die Relaxationszeit weniger als 119 ms, vorzugsweise weniger als 80 ms.

Als besonders vorteilhaft für eine effektivere transdermale Freisetzung hat sich ein abgestimmtes Verhältnis der Komponenten (Strukturbrecher/Strukturbildner) erwiesen.

Erfindungsgemäß beträgt das Verhältnis zwischen Strukturbrecher und Strukturbildner 10:1 bis 1:10, bevorzugterweise beträgt das Verhältnis zwischen Strukturbrecher und Strukturbildner 2:1 bis 1:2.

Als Strukturbrecher wird eine Substanz verwendet, welche die Wasserstruktur zerstört, so daß eine Relaxationszeit > 120 ms, vorzugsweise > 150 ms resultiert. Diese höhere Relaxationszeit ist Ausdruck einer größeren Beweglichkeit freien Wassers, das damit für Lösungs- und Diffusionsprozesse zur Verfügung steht.

Als Strukturbildner wird eine Substanz verwendet, welche die Wasserstruktur verfestigt, so daß eine Relaxationszeit < 120 ms, vorzugsweise < 80 ms resultiert. Diese geringere Relaxationszeit ist Ausdruck einer verminderten Beweglichkeit freien Wassers, das damit für Lösungs- und Diffusionsprozesse nicht mehr zur Verfügung steht.

Den dargelegten Sachverhalt verdeutlicht eine Untersuchung der $T_2$ Relaxationszeiten für ausgewählte Strukturbrecher und Strukturbildner (Tabelle 2).

Tabelle 2

| $T_2$ Relaxationszeiten für ausgewählte Strukturbrecher und Strukturbildner in Wasser (Konzentration: 0,5 mol/l) | | | |
|---|---|---|---|
| Gelöstes | Art | $T_2$ [ms] ± SEM [ms] | |
| - | Wasser | 129 | 20 |
| Saccharose | Strukturbildner | 18,7 | 0,1 |
| Sorbitol | Strukturbildner | 119,4 | 14 |
| Harnstoff | Strukturbrecher | 145 | 15 |
| Nicotinamid | Strukturbrecher | 276 | 24 |

Das geeignete Verhältnis der beiden Substanzen wurde in einer breit angelegten Versuchsreihe mit Testosteron und anderen Steroidhormonen ermittelt. Dabei zeigte sich überraschend, daß eine additive Steigerung des transdermalen Fluxes erreicht werden kann, wenn nicht nur die Struktur des Wassers gebrochen wird, sondern zugleich durch Verwendung einer festen Dispersion des Wirkstoffes in der Zubereitung mehr Wirkstoff für die Lösung und Diffusion angeboten wird.

Überaschenderweise wird durch das erfindungsgemäße transdermale therapeutische System eine erhebliche Verbesserung des transdermalen Fluxes erreicht. Dieser Wert beträgt in den untersuchten Fällen mindestens das 1,4-fache herkömmlicher TTS.

Sollte aus therapeutischen Erfordernissen eine genaue Einstellung des Fluxes gewünscht sein, so kann dies

erfindungsgemäß durch die gleichzeitige Verwendung von Strukturbrechern und Strukturbildnern in einem abgemessenen Verhältnis erreicht werden.

Ferner wird eine erfindungsgemäße Zusammensetzung zur transdermalen Verabreichung bereitgestellt, die in einer Menge von mindestens mehr als 10 Gew.-% und weniger als 90 Gew.-% ein penetrationsverstärkendes Agens der nachfolgenden Formel 1

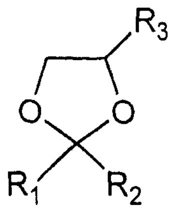

(1)

enthält, in der R1 und R2 identisch oder verschieden sind und ausgewählt sind aus der Gruppe der C1- bis C6-Alkylreste, insbesondere der wahlweise verzweigten, gesättigten C1- bis C4-Alkylreste, R3 ausgewählt ist aus der Gruppe, bestehend aus Hydroxy-(C1- bis C6-)Alkylresten, insbesondere Hydroxy-(C1- bis C4-) Alkylresten sowie mindestens einem Wirkstoff oder dessen pharmazeutisch akzeptablen Salz und ein weiteres lipophiles penetrationsverstärkendes Agens, enthält.

Außerdem wird erfindungsgemäß eine weitere Zusammensetzung zur transdermalen Verabreichung bereitgestellt. Diese umfaßt ein penetrationsverstärkendes Agens der vorstehend genannten Formel 1, wobei R1, R2 und R3 wie vorstehend definiert sind, mindestens einen Wirkstoff oder dessen pharmazeutisch akzeptables Salz und ein weiteres lipophiles penetrationsverstärkendes Agens, ausgenommen Ölsäure, dessen Lipophilie, gemessen mittels Bestimmung des Wasseraufnahmevermögens, im Bereich von 0 - 1,4 Gew.-%, bevorzugt im Bereich von 0,001 - 0,330 Gew.-% oder im Bereich von 0,340 - 1,400 Gew.-% liegt.

Gegenstand der vorliegenden Erfindung ist somit eine Zusammensetzung zur transdermalen Verabreichung, umfassend in einer Menge von mindestens mehr als 10 Gew.-% und weniger als 90 Gew~% ein penetrationsverstärkendes Agens der nachfolgenden Formel 1

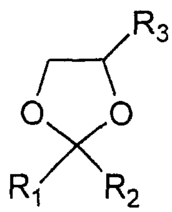

(1)

wobei R1 und R2 identisch oder verschieden sind und ausgewählt sind aus der Gruppe der C1- bis C6-Alkylreste, insbesondere der wahlweise verzweigten, gesättigten C1-bis C4-Alkylreste,
R3 ausgewählt ist aus der Gruppe, bestehend aus Hydroxy-(C1- bis C6-)Alklyresten, insbesondere Hydroxy-(C1 - bis C4-)Alkylresten

- sowie mindestens einen pharmazeutischen Wirkstoff oder dessen pharmazeutisch akzeptables Salz und
- ein weiteres lipophiles penetrationsverstärkendes Agens.

Gegenstand der vorliegenden Erfindung ist ferner eine Zusammensetzung zur transdermalen Verabreichung, umfassend ein penetrationsverstärkendes Agens der nachfolgenden Formel 1

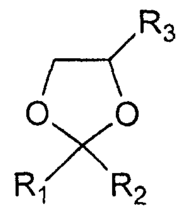

(1)

wobei R1 und R2 identisch oder verschieden sind und ausgewählt sind aus der Gruppe der C1- bis C6-Alkylreste, insbesondere der wahlweise verzweigten, gesättigten C1-bis C4-Alkylreste
R3 ausgewählt ist aus der Gruppe, bestehend aus Hydroxy-(C1- bis C6-)Alkylresten, insbesondere Hydroxy-(C1- bis C4-)Alkylresten

- sowie mindestens einen pharmazeutischen Wirkstoff oder dessen pharmazeutisch akzeptables Salz und

- ein weiteres lipophiles penetrationsverstärkendes Agens, ausgenommen Ölsäure, dessen Lipophilie, gemessen mittels Bestimmung des Wasseraufnahmevermögens, im Bereich von 0 bis 1,4 Gew.-%, bevorzugt im Bereich von 0,001 bis 0,330 Gew.-% oder im Bereich von 0,340 bis 1,400 Gew.-% liegt.

Bevorzugt ist eine Zusammensetzung, wobei das penetrationsverstärkende Agens der Formel 1 in einem Anteil von mehr als 10 bis 50 Gew.-%, bevorzugt mehr als 10 bis 25 Gew.-%, am allerbevorzugtesten 15 bis 25 Gew.-% enthalten ist.
Bevorzugt ist auch eine Zusammensetzung, wobei das penetrationsverstärkende Agens der Formel 1 Solketal (2,2-Dimethyl-4-hydroxymethyl-1,3-dioxolan) ist.
Weiterhin bevorzugt ist eine Zusammensetzung, desweiteren enthaltend einen Haftkleber, bevorzugt einen Haftkleber auf der Basis von Mischungen, enthaltend (Co-)Polymere auf der Basis von Bestandteilen, welche ausgewählt sind aus der Gruppe von C1- bis C6-Alkyl-(meth)acrylaten, C1-bis C5-hydroxyalkyl-(meth)acrylaten, insbesondere enthaltend einen Haftkleber auf der Basis von Mischungen, enthaltend (Co-)Polymere auf der Basis von Bestandteilen, ausgewählt aus der Gruppe, bestehend aus Vinylacetat, 2-Ethylhexylacrylat, 2-Hydroxyethylacrylat, Glycidylmethacrylat, Butylacrylat, Acrylsäure und Methylacrylat.
Außerdem bevorzugt ist eine Zusammensetzung, wobei das weitere lipophile penetrationsverstärkende Agens ausgewählt ist aus der Gruppe der gesättigten Kohlenwasserstoffe mit 10 bis 30 C-Atomen, der wahlweise ungesättigten Fettalkohole mit 10 bis 30 C-Atomen, der gesättigten oder ungesättigten, ein- oder mehrwertigen Fettsäuren mit 8 bis 30 C-Atomen und deren Ester mit wahlweise ungesättigten Fettalkoholen mit 10 bis 30 C-Atomen sowie Triacylglyceriden mit Fettsäureresten mit 8 bis 22 C-Atomen, bevorzugt mit 5 bis 12 C-Atomen.
Insbesondere bevorzugt ist dabei eine Zusammensetzung, wobei das weitere lipophile penetrationsverstärkende Agens ausgewählt ist aus der Gruppe, bestehend aus Dioctylcyclohexan, Dodecanol, 2-Octyldodecanol, 2-Hexyldodecanol, Oleylalkohol, Laurinsäure, Ölsäure, Palmitinsäure, Dioctylether, Isopropylmyristat, Hexyllaurat, Cetearyl-isononanoat. Caprinsäure, Caprinsäure-(C1 - bis C20-)alkylester, Ölsäure-(C1- bis C20-)alkylester, insbesondere Decyloleat, Oleyloleat, Docosensäure-(C1- bis C20-)alkylester.
Ganz besonders bevorzugt ist hierbei eine Zusammensetzung, wobei das weitere lipophile penetrationsverstärkende Agens Ethyloleat ist.
Bevorzugt ist ferner eine Zusammensetzung, wobei das weitere lipophile penetrationsverstärkende Agens in einer relativen Menge von mindestens 2 Gew-%, bevorzugt zwischen 5 und 15 Gew.-% enthalten ist.
Bevorzugt ist auch eine Zusammensetzung, wobei ein weiteres hydrophiles penetrationsverstärkendes Agens enthalten ist, das bevorzugt eine Wasserlöslichkeit von mindestens 5 Gew.-% aufweist.
Besonders bevorzugt ist hierbei eine Zusammensetzung, wobei das weitere hydrophile penetrationsverstärkende Agens ausgewählt ist aus der Gruppe, bestehend aus Säureamiden, Polyethylenglykolen, Glykolen, Pyrrolidonen, Polymeren von Pyrrolidonderivaten, insbesondere Nicotinsäureamid oder Harnstoff.
Ganz besonders bevorzugt ist eine Zusammensetzung, wobei das weitere hydrophile penetrationsverstärkende Agens in einer relativen Menge von 1 bis 10 Gew.-%, bevorzugt 2 bis 5 Gew.-% enthalten ist.
Ferner bevorzugt ist eine Zusammensetzung, wobei es sich bei dem Wirkstoff um einen lipophilen Wirkstoff1 insbesondere ein steroidales Agens handelt.
Besonders bevorzugt ist hierbei eine Zusammensetzung, wobei das steroidale Agens ein Steroidhomon, insbesondere ausgewählt aus der Gruppe, bestehend aus Corticosteroiden, Sexualhormonen, bevorzugt Estrogene, Gestagene, Androgene, ist, insbesondere aus der Gruppe, bestehend aus Testosteron, Estradiol und deren Derivaten,

besonders bevorzugt Testosteron, Estradiol, Ethinylestradiol und Norethisteronacetat.

Außerdem bevorzugt ist eine Zusammensetzung, wobei die Zusammensetzung den Wirkstoff in zumindest gesättigter Lösung, vorzugsweise in übersättigter Lösung enthält.

Die in dem erfindungsgemäßen transdermalen therapeutischen System enthaltenen Wirkstoff können nahezu beliebig ausgewählt werden. Bevorzugte erfindungsgemäße Wirkstoffe sind aus Hormonen, Immunmodulatoren, Immunsuppressiva, Antibiotika, Cytostatika, Diuretika, Magen-Darm-Mittel, Herz-Kreislauf-Mittel und Neuropharmaka oder deren Mischungen ausgewählt.

Besonders bevorzugt sind Hormone, insbesondere Sexualhormone, wie beispielsweise Testosteron, Estradiol, Estriol, Norethisteron, Dienogest oder deren Mischungen.

Die erfindungsgemäßen transdermalen therapeutischen Systeme lassen sich mit einfachen Verfahren herstellen. Diese Verfahren basieren im wesentlichen auf bekannten pharmazeutischen Technologien. Das Verfahren kommt grundsätzlich ohne eine aufwendige Präzisionsfertigung oder spezielle Überzugstechniken aus. Das Verfahren liefert Arzneiformen, aus denen heraus hydrophile Arzneistoffe verbessert die Haut durchdringen.

Dabei werden die Wirkstoffe erfindungsgemäß in Form fester Dispersionen verwendet. Entsprechende Dispersionen sind herstellbar beispielsweise nach der DE-OS 44 02 462.

Gegenstand der vorliegenden Erfindung sind ferner Mittel zur transdermalen Verabreichung, umfassend die oben beschriebene erfindungsgemäße Zusammensetzung.

Besonders bevorzugt ist dabei ein Mittel, wobei das Mittel eine Emulsion, Salbe, Creme, Lotion oder transdermales therapeutisches System (TTS) ist.

Besonders bevorzugt ist hierbei ein TTS, mit einer gegebenenfalls abziehbaren Schutzschicht (1), mindestens einer haftklebenden Matrixschicht, insbesondere einer haftkleberhaltigen Grundierschicht (2) sowie einer haflkleberhaltigen Hautschicht (3), einer weiteren Zwischenschicht (4) und einer wahlweise wirkstoffund/oder wasserdampfundurchlässigen Rückschicht (5).

Insbesondere bevorzugt ist ein TTS, wobei die Zwischenschicht (4) von der Grundierschicht (2) und der Hautschicht (3) allseitig überragt wird.

Bevorzugt ist dabei auch ein TTS, wobei die Zusammensetzung gegebenenfalls auf drei Kompartimente aufgeteilt vorliegt, nämlich die haftkleberhaltige Grundierschicht (2), die haftkleberhaltige Hautschicht (3) und die weitere Zwischenschicht (4).

Erfindungsgemäß ist die Matrix des transdermalen therapeutischen Systems ein flächenförmiges klebendes Gebilde, ein Pflaster, ein Patch, ein Gel, eine Salbe, eine Creme, eine Emulsion, eine Einreibung, eine Pinselung oder ein getränktes Gewebe.

Gegenstand der vorliegnden Erfindung sind somit nicht nur die bekannten flächenförmigen klebenden Gebilde (Pflaster, "Patches") sondern auch Gele, Salben, Cremes, Emulsionen, Einreibungen, Pinselungen, getränkte Gewebe und ähnliche Applikationsvorrichtungen transdermaler therapeutischer Systeme, sofern sie auf dem Zielorgan Haut oder Schleimhaut haften.

Gegenstand der Erfindung ist ferner ein Verfahren zur Herstellung des oben beschriebenen erfindungsgemäßen TTS, umfassend die Schritte, daß man

- ein erstes Laminat herstellt, indem man eine erste Mischung enhaltend den mindestens einen Wirkstoff und einen wahlweise quervernetzbaren Haftkleber herstellt und auf einem Schutzschicht (1) -Trägermaterial aufbringt,

- ein zweites Laminat herstellt, indem man die erste Mischung auf einem Rückschicht (5) -Trägermaterial aufbringt,

- aus einem weiteren Trägermaterial, bevorzugt einem Vlies, eine Formscheibe ausstanzt und auf dem ersten Laminat aufbringt,

- und eine zweite Mischung, enthaltend das penetrationsverstärkende Agens gemäß Formel 1 und das weitere lipophile penetrationsverstärkende Agens, auf dem weiteren Trägermaterial aufbringt,

- das zweite Laminat darüberlaminiert und Einzel-TTS daraus herstellt.

Die Herstellung des TTS beruht ansonsten auf der grundsätzlich bekannten pharmazeutischen Technologie dieser Systeme und ist somit in den dafür üblichen pharmazeutischen Produktionseinrichtungen ohne besondere Vorkehrungen durchführbar.

Gegenstand der Erfindung ist schließlich ein nach diesem Verfahren hergestelltes Erzeugnis zur Anwendung in der Substitutionstherapie, insbesondere Hormonsubstitutionstherapie.

Bevorzugt ist dabei ein Erzeugnis zur Anwendung bei Hypogonadismus, Anämie, angeborenem Angiödem, Impotenz, Unfruchtbarkeit oder Empfängnisverhütung.

Die Erfindung wird durch die nachfolgenden Beispiele näher erläutert.

**Untersuchungsmethode 1**

Die transdermale Permeation der entsprechenden Arzneistoffe durch exzidierte Rindereuterhaut (Schichtdicke 1,2 mm) wurde in einer modifizierten FRANZ-Zelle (Hansen-Research) untersucht. Zu Beginn des Versuchs wurden die verwendeten Vehikel ins Donor-Kompartiment der Zelle eingebracht und über einen Zeitraum von 56 h die permeierte Masse Wirkstoff m durch vollständige Entnahme und Austausch des Akzeptor-Kompartiments nach t = 8, 24, 32, 48 und 56 Stunden durch UV- bzw. fluoreszenzspektroskopische Detektion und HPLC-Trennung quantifiziert. Die m/t-Profile im Zeitabschnitt 24 h $\leq$ t $\leq$ 56 h wurden linearisiert, so daß aus dem Anstieg der Regressionsgeraden der in vitro Flux J berechnet wurde.

Beispiel 1

Testosteron-Hydrogel 0,5 %
Folgendes Hydrogel wurde verwendet:

| | |
|---|---|
| Testosteron | 0,500 g |
| Carbopol ® 934 | 0,500 g |
| Triethanolamin | 0,500 g |
| Ethanol 96% | 46,875 g |
| Aqua purificata | ad 100,000 g |

Dem Hydrogel wurden die in Tabelle 3 aufgeführten Strukturbrecher/-bildner in entsprechender Konzentration zugefügt und der Wasseranteil um die entsprechende Masse reduziert.
Die transdermalen in vitro-Flux-Werte J der Hydrogele, die Standardabweichung S und die entsprechenden Enhancement-Faktoren bei Anwesenheit eines Strukturbrechers/ -bildners sind in Tabelle 3 dargestellt.

Tabelle 3

| Flux J und Enhancement-Faktor $F_E$ der Testosteron-Hydrogele mit und ohne Zusatz von Strukturbrechern/bildnern | | | | |
|---|---|---|---|---|
| Strukturbrecher | Strukturbildner | Konzentration | Flux J ± S [$\mu g/cm^2*h$] | $F_E$ |
| / | / | / | 3,1 ± 1,1 | |
| Nicotinamid | / | 0,5 mol/kg | 5,5 ± 1,2 | 1,75 |
| Harnstoff | / | 0,5 mol/kg | 6,9 ± 2,3 | 2,22 |
| / | Lactose | 45 g/kg | 3,7 ± 0,7 | 1,19 |
| / | Lactose (feste Dispersion) | 45 g/kg | 7,4 ± 0,7 | 2,39 |
| Nicotinamid | Lactose (feste Disp.) | 0,5 mol/kg/ 45 g/kg | 11,8 ± 2,4 | 3,8 |

Der gefundene Flux J beträgt zwischen 3,1 ± 1,1 $\mu g/cm^2*h$ und 11,8 ± 2,4 $\mu g/cm^2*h$. Die Enhancement-Faktoren bei Zugabe von Strukturbrechern/-bildnern betragen zwischen 1,75 und 3,8. Somit konnte gezeigt werden, daß durch die Zugabe von Strukturbrechern und/oder Strukturbildnern in festen Dispersionen mit dem Wirkstoff der in vitro-Flux von Wirkstoffen gesteigert werden kann. Eine Kombination beider "Enhancer" steigert den Flux additiv.

Beispiel 2

Estriol-Creme 4 %
Folgende Creme wurde verwendet:

| | |
|---|---|
| Estriol | 4,000 g |
| Konservierungsmittel | 0,025 g |
| Propylenglycol | 2,000 g |
| Silikonöl | 2,000 g |

(fortgesetzt)

| | |
|---|---|
| Glycerolmonostearat, selbstemulgierend | 5,000 g |
| Triglyceride, mittelkettig | 5,000 g |
| Fettphase | 35,200 g |
| Gereinigtes Wasser | 46,775 g |

Dem Hydrogel wurden die in Tabelle 4 aufgeführten Strukturbrecher/-bildner in entsprechender Konzentration zugefügt und der Wasseranteil um die entsprechende Masse reduziert.

Die transdermalen in vitro-Flux-Werte J der Cremes, die Standardabweichung S und die entsprechenden Enhancement-Faktoren $F_E$ bei Abwesenheit eines Strukturbrechers/bildners sind in Tabelle 4 dargestellt.

Tabelle 4

| Flux J und Enhancement-Faktor $F_E$ der Estriol-Cremes mit und ohne Zusatz von Strukturbrechern/ -bildnern | | | | |
|---|---|---|---|---|
| Strukturbrecher | Strukturbildner | Konzentration | Flux J ± S [µg/cm$^2$*h] | $F_E$ |
| / | / | / | 0,184 + 0,023 | / |
| Nicotinamid | / | 1 mol/l | 0,508 ± 0,060 | 2,76 |

Der gefundene Flux beträgt zwischen 0,184 ± 0,023 µg/cm$^2$*h und 0,508 ± 0,060 µg/cm$^2$*h. Der Enhancement-Faktor bei Zugabe von Nicotinamid beträgt 2,76. Somit konnte gezeigt werden, daß durch die Zugabe von Strukturbrechern der in vitro-Flux von Wirkstoffen gesteigert werden kann.

Beispiel 3

Dienogest-Hydrogel 0,5 %

Folgendes Hydrogel wurde verwendet:

| | |
|---|---|
| Dienogest | 0,500 g |
| Carbopol ® 934 | 0,500 g |
| Triethanolamin | 0,500 g |
| Ethanol 96% | 46,875 g |
| Aqua purificata | ad 100,000 g |

Dem Hydrogel wurden die in Tabelle 5 aufgeführten Strukturbrecher/ -bildner in entsprechender Konzentration zugefügt und der Wasseranteil um die entsprechende Masse reduziert.

Die transdermalen in vitro-Flux-Werte J der Hydrogele, die Standardabweichung S und die entsprechenden Enhancement-Faktoren bei Anwesenheit eines Strukturbrechers/- bildners sind in Tabelle 5 dargestellt.

Tabelle 5

| Flux J und Enhancement-Faktor $F_E$ der Dienogest-Hydrogele mit und ohne Zusatz von Strukturbrechern/ -bildnern | | | | |
|---|---|---|---|---|
| Strukturbrecher | Strukturbildner | Konzentration | Flux J ± S [µg/cm$^2$*h] | $F_E$ |
| / | / | / | 1,38 ± 0,27 | |
| Nicotinamid | Lactose (feste Dispersion) | 0,5 mol/kg 45 g/kg | 1,97 ± 0,26 | 1,43 |

Der gefundene Flux J beträgt zwischen 1,38 ± 0,27 g/cm$^2$*h und 1,97 ± 0,26 µg/cm$^2$*h. Die Enhancement-Faktoren bei Zugabe von Strukturbrechern/ -bildnern beträgt 1,43. Somit konnte gezeigt werden, daß durch die Zugabe von Strukturbrechern und/oder Strukturbildnern in festen Dispersionen mit dem Wirkstoff der in vitro-Flux von Wirkstoffen gesteigert werden kann.

**Untersuchungsmethode 2**

Studiendesign:

Es wurde eine offene randomisierte dreiarmige Cross-over-Studie durchgeführt, dessen einer Arm das Testosteron-TTS darstellte. Dieses TTS soll 2,5 mg Testosteron über 24 Stunden kontrolliert freisetzen. Als Testpersonen wurden 9 postmenopausale bzw. menopausale Frauen im Alter von 20 bis 65 Jahren ausgewählt.
Das Testosteron-TTS wurde zur Zeit $t_0$ auf den Unterarm aufgebracht und nach 24 Stunden entfernt. Das Serum wurde zu bestimmten Zeiten entnommen und hinsichtlich der Testosteron-Konzentration mit RIA analysiert. Die letzte Entnahme erfolgte 24 Stunden nach Entfernen des TTS.

Beispiel 4

Das untersuchte Testosteron-TTS war wie in Tabelle 6 dargestellt zusammengesetzt.

Tabelle 6

| Zusammensetzung des Testosteron-TTS | | |
|---|---|---|
| **Rohstoff** | **Gehalt/TTS** | **% in der Matrix** |
| Testosteron | 8,4 mg | 3,00 |
| Nicotinamid | 14,0 mg | 5,00 |
| Matrix | 257,6 mg | 92,00 |
| Backing Layer | 35,0 $cm^2$ | |
| Release Liner | 46,2 $cm^2$ | |

In Tabelle 7 sind die mittleren Testosteron-Serum-Konzentrationen des Testosteron-TTS der Pilotstudie zusammengestellt.

Tabelle 7

| Testosteron-Serum-Konzentrationen c des Testosteron-TTS in Abhängigkeit von der Zeit t | | | | | | | | |
|---|---|---|---|---|---|---|---|---|
| t [h] | 1 | 2 | 3 | 4 | 5 | 7 | 8 | 10 |
| c [ng/ml] | 0,01 | 0,05 | 0,15 | 0,24 | 0,62 | 0,65 | 0,77 | 0,90 |
| t [h] | 12 | 16 | 24 | 25 | 26 | 28 | 32 | 48 |
| c [ng/ml] | 1,05 | 1,25 | 1,15 | 0,82 | 0,58 | 0,33 | 0,14 | 0,10 |

Figur 1 zeigt die mittleren Serumkonzentrationen der untersuchten Probandinnen (n = 8) von Testosteron über einen Zeitraum von 48 Stunden. Die Serumkonzentrationen liegen im Bereich von 0,01 ng/ml nach 1 Stunde und 1,25 ng/ml nach 16 Stunden.
Es ist nach einer kurzen lag-Zeit von ca. 2 Stunden ein deutlicher Anstieg der Serumkonzentrationen zu erkennen, der im Bereich von über 1 ng/ml über 12 Stunden konstant bleibt. 24 Stunden nach Aufbringen des TTS fällt die Serumspiegelkurve relativ jäh ab. Das bedeutet, daß nach etwa 24 Stunden der überwiegende Teil des Testosterons aus dem TTS herausdiffundiert und durch die Haut permeiert ist.

**Untersuchungsmethode 3**

Studiendesign:

Es wurde eine offene randomisierte dreiarmige Cross-over-Studie mit "single dose" Applikation durchgeführt. Die drei Arme waren drei Estriol Cremes gleicher Grundlage und unterschiedlicher Dosierung des Wirkstoffs. Es sollte die Bioverfügbarkeit von Estriol nach transdermaler Applikation festgestellt werden. Als Testpersonen wurden 9 postmenopausale Frauen ausgewählt. Die entsprechenden Estriol Cremes wurden zur Zeit $t_0$ auf den Unterarm aufgetragen. Das Serum wurde zu bestimmten Zeiten in einem Zeitraum bis 24 Stunden entnommen und hinsichtlich der Estriol-Konzentration mit RIA analysiert. Die letzte Entnahme erfolgte 24 Stunden nach Applikation der Cremes.

Beispiel 5

Estriol Cremes

Die Zusammensetzung der untersuchten Cremes ist in Tabelle 8 zusammengestellt.

Tabelle 8

| Zusammensetzung der Estriol Cremes 1, 3 und 10 | | | |
|---|---|---|---|
| **Wirk- und Hilfsstoffe** | **Estriol 1** | **Estriol 3** | **Estriol 10** |
| Estriol | 1,000 g | 3,000 g | 10,000 g |
| Konservierungsmittel | 0,025 g | 0,024 g | 0,022 g |
| Propylenglycol | 1,980 g | 1,940 g | 1,800 g |
| Silikonöl | 1,980 g | 1,940 g | 1,800 g |
| Glycerolmonostearat, selbstemulgierend | 4,950 g | 4,850 g | 4,500 g |
| Triglyceride, mittelkettig | 4,950 g | 4,850 g | 4,500 g |
| Fettphase | 34,848 g | 34,144 g | 31,680 g |
| Nicotinsäureamid | 6,039 g | 5,917 g | 5,490 g |
| gereinigtes Wasser | 44,228 g | 43,335 g | 40,208 g |

Figur 2 zeigt die mittleren Estriol-Serumkonzentrationen der untersuchten Probandinnen (n = 8) über einen Zeitraum von 24 Stunden. Die Serumkonzentrationen liegen im Bereich von 1,09 pg/ml nach 0 Stunden und 222,04 pg/ml nach 24 Stunden (jeweils Estriol Creme 10).

Es ist nach einer lag-Zeit von ca. 10 - 12 Stunden ein deutlicher Anstieg der Serumkonzentrationen zu erkennen. Sie liegen bei der Estriol Creme 10 am höchsten und bei der Estriol Creme 1 am niedrigsten. Es ist eine Konzentrations- bzw. Dosisabhängigkeit der Serumkonzentrationen zu erkennen. Da die Serumkonzentrationen bis 24 Stunden nach Applikation noch bei allen drei Cremes ansteigen, ist ein weiterer Anstieg der Serumkonzentrationen zu erwarten.

**Untersuchungsmethode 4**

Zur Überprüfung des Wirkprinzips der Strukturbrecher wurden NMR-Experimente an einem Biospec 47/40 der Firma Brucker durchgeführt (Fraunhofer-Institut für Biomedizinische Technik, D-St. Ingbert). Die Feldstärke betrug 4,7 T. Zur Sicherheit wurden in jeweils einem Experiment (Repetitionszeit 1,5 s) gleichzeitig 3 koronale, aneinander angrenzende Schnitte für das vordere Glied des Mittelfingers von Probanden aufgenommen. Für den Schnitt wurden 8 Echobilder im zeitlichen Abstand von 8 ms gemessen (Hermite-Impulse, Dauer 1 ms), so daß 24 Bilder resultierten. Die Dicke der selektierten Schnitte betrug 2 mm. Für jedes Bild wurden 256*256 Pixel aufgenommen, die ein Sichtfeld von 3 cm abdeckten. Damit wurde eine Auflösung in der Bildebene von 120 um gewährleistet. Zur Auswertung diente die Software TOMIKON.

Beispiel 6

Hydrogel mit 0,5 mol/kg Nicotinamid

Den Probanden wurde ein Gel mit dem erfinderischen Prinzip (vgl. Beispiel 1) im Vergleich zu einem Gel ohne Zusätze appliziert (aus arzneimittelrechtlichen Gründen wurde mit wirkstoffreien Gelen gearbeitet).

Die Untersuchung zeigte

1. anhand der Protonenspindichte eine Beeinflussung des Wassers (wahrscheinlich in der Region B) durch das erfindungsgemäße Prinzip,

2. anhand der $T_2$ Relaxionszeiten eine deutliche Beeinflussung der äußeren Hautschichten durch das erfindungsgemäße Prinzip.

Hier wurden in einer in vitro Studie für ein ethanolisches Hydrogel ohne Nicotinamid-Zusatz ein $T_2$-Wert von 46,4 ms gefunden. Der Zusatz von 1 M Nicotinamid führte zu einem $T_2$-Wert von 61,2 ms, wohingegen eine Darstellung des Gels unter Nicotinamid-Zusatz mit der "HSP"-Technik (feste Dispersion von Wirkstoff und Lactose) zu einer Verkürzung der $T_2$ Relaxationszeit von 34,0 ms führte.

Zusammenfassend läßt sich das Ergebnis der Untersuchungen wie folgt zusammenfassen:

- Durch einen Strukturbrecher wird freies Wasser für die Lösung und Diffusion zur Verfügung gestellt. Dabei läßt sich das Ausmaß dieses Prozesses durch die Art und/oder Konzentration des Strukturbrechers und/oder durch die Abmischung mit einem Strukturbildner sehr genau den jeweiligen Erfordernissen anpassen.

- Der Wirkstoff kann, wenn er als feste Dispersion vorliegt, in dem freien Wasser schnell in Lösung gehen und diffundieren.

**Untersuchungsmethode 5**

Studiendesign:

Es wurde eine offene randomisierte vierarmige Cross-over-Studie durchgeführt, deren einer Arm ein Testosteron-TTS nach Beispiel 7 darstellte. Dieses TTS soll 3,5 mg Testosteron über 24 Stunden kontrolliert freisetzen. Als Testpersonen wurden 8 post- bzw. menopausale Frauen im Alter von 20 bis 65 Jahren ausgewählt.

Das Testosteron-TTS wurde zur Zeit $t_0$ auf den Unterarm aufgebracht und nach 24 Stunden entfernt. Den Frauen wurde Blut zu bestimmten Zeiten entnommen, undim Serum wurde die Testosteron-Konzentration mittels RIA analysiert. Die letzte Blutentnahme erfolgte 10 Stunden nach Entfernen des TTS.

Beispiel 7

Das untersuchte Testosteron-TTS war wie in Tabelle 9 dargestellt zusammengesetzt.

Tabelle 9

| Zusammensetzung des Testosteron-TTS | | |
|---|---|---|
| **Rohstoff** | **Gehalt/TTS** | **% in der Matrix** |
| Testosteron | 21,0 mg | 3,50 |
| Nicotinamid | 21,0 mg | 3,50 |
| Matrixkomponenten | 556,32 mg | 93,00 |
| Backing Layer | 35,0 $cm^2$ | |
| Release Liner | 46,2 $cm^2$ | |

In Tabelle 10 sind die mittleren Testosteron-Serum-Konzentrationen des Testosteron-TTS zusammengestellt.

Tabelle 10

| Testosteron-Serum-Konzentrationen c des Testosteron-TTS in Abhängigkeit von der Zeit t | | | | | | | | |
|---|---|---|---|---|---|---|---|---|
| t [h] | 0 | 1,5 | 3 | 5 | 7 | 9 | 12 | 16 |
| c [ng/ml] | 0 | 0,04 | 0,40 | 0,78 | 1,40 | 1,57 | 1,88 | 2,20 |
| t [h] | 24 | 25 | 26 | 27 | 28 | 30 | 32 | 34 |
| c [ng/ml] | 2,00 | 1,29 | 1,21 | 0,98 | 0,86 | 0,76 | 0,43 | 0,24 |

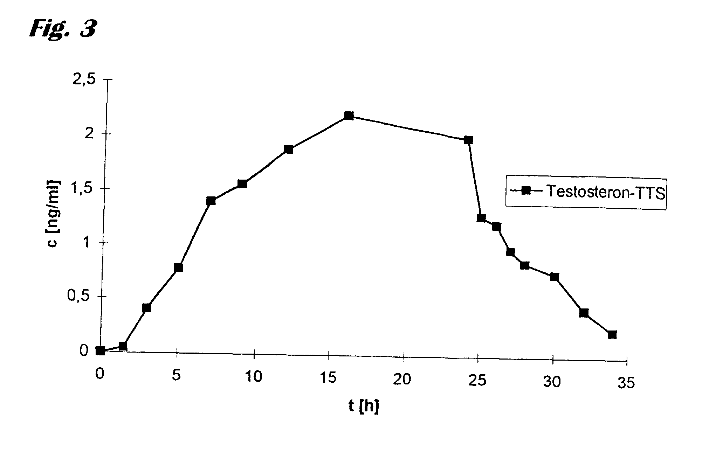

Figur 3 zeigt die mittleren Serumkonzentrationen (n=8) von Testosteron über einen Zeitraum vom 34 Stunden. Die Serumkonzentrationen liegen im Bereich von 0,04 ng/ml nach 1,5 Stunden und 2,20 ng/ml nach 16 Stunden. Es ist nach einer kurzen lag-Zeit von ca. 2 Stunden ein deutlicher Anstieg der Serumkonzentrationen zu erkennen, der im Bereich von über 1 ng/ml über 20 Stunden bleibt. 24 Stunden nach Aufbringen des TTS, zeitgleich mit dem Entfernen des TTS, fällt die Serumspiegelkurve kontinuierlich ab.

## Patentansprüche

1. Transdermales therapeutisches System zur Anwendung auf der Haut und/oder Schleimhaut bestehend aus mindestens einem Wirkstoff in Form einer festen Dispersion in Kombination mit mindestens einem Strukturbrecher, bei dessen alleiniger Verwendung die Relaxationszeit des transdermalen therapeutischen Systems mehr als 120 ms betragen würde, und/oder mindestens einem Strukturbildner, bei dessen alleiniger Verwendung die Relaxationszeit des transdermalen therapeutischen Systems weniger als 119 ms betragen würde, in einer gemeinsamen Matrix.

2. Transdermales therapeutisches System gemäß Anspruch 1, **dadurch gekennzeichnet, daß** die feste Dispersion eine molekulardisperse Verteilung des Wirkstoffs in einer inerten Trägersubstanz ist.

3. Transdermales therapeutisches System gemäß Anspruch 2, **dadurch gekennzeichnet, daß** die inerte Trägersubstanz aus an sich bekannten inerten Trägerstoffen für feste Dispersionen, wie Saccharose, Lactose, Bernsteinsäure Polyethylenglycolen, Polyvinylpyrrolidon, Harnstoff, Mannitol, Mannitose oder deren Mischungen ausgewählt ist.

4. Transdermales therapeutisches System gemäß einem der Ansprüche 1 bis 3, **dadurch gekennzeichnet, daß** der Strukturbrecher aus der Gruppe der Carbonsäureamide, wie Harnstoff, Nicotinsäureamid, Bernsteinsäureamid, Methylacetamid, Ethylacetamid oder deren Mischungen ausgewählt ist.

5. Transdermales therapeutisches System gemäß Anspruch 4, **dadurch gekennzeichnet, daß** die Relaxationszeit mehr als 120 ms, vorzugsweise mehr als 150 ms beträgt.

6. Transdermales therapeutisches System nach einem der Ansprüche 1 bis 5, **dadurch gekennzeichnet, daß** der Strukturbildner aus der Gruppe der Polyole, wie beispielsweise Glycerin, Ethylenglycol, Propylenglycol, aus der Gruppe der Zuckeralkohole, wie beispielsweise Sorbitol und/oder aus der Gruppe der Zucker, wie beispielsweise Saccharose oder Glucose oder deren Mischungen ausgewählt ist.

7. Transdermales therapeutisches System gemäß Anspruch 6, **dadurch gekennzeichnet, daß** die Relaxationszeit weniger als 119 ms, vorzugsweise weniger als 80 ms beträgt.

8. Transdermales therapeutisches System gemäß einem der voranstehenden Ansprüche, **dadurch gekennzeichnet, daß** das Verhältnis zwischen Strukturbrecher und Strukturbildner 10:1 bis 1:10 beträgt.

9. Transdermales therapeutisches System gemäß Anspruch 8, **dadurch gekennzeichnet, daß** das Verhältnis zwischen Strukturbrecher und Strukturbildner 2:1 bis 1:2 beträgt.

10. Transdermales therapeutisches System gemäß einem der voranstehenden Ansprüche, **dadurch gekennzeichnet, daß** der Wirkstoff aus Hormonen, Immunmodulatoren, Immunsuppressiva, Antibiotika, Cytostatika, Diuretika, Magen-Darm-Mittel, Herz-Kreislauf-Mittel und Neuropharmaka oder deren Mischungen ausgewählt ist.

11. Transdermales therapeutisches System gemäß Anspruch 10, **dadurch gekennzeichnet, daß** der Wirkstoff aus Hormonen, wie Testosteron, Estradiol, Estriol, Norethisteron, Dienogest oder deren Mischungen ausgewählt ist.

12. Transdermales therapeutisches System einem der Ansprüche 1 bis 11, **dadurch gekennzeichnet, daß** die Matrix ein flächenförmiges klebendes Gebilde, ein Pflaster, ein Patch, ein Gel, eine Salbe, eine Creme, eine Emulsion, eine Einreibung, eine Pinselung oder ein getränktes Gewebe ist.

13. Zusammensetzung zur transdermalen Verabreichung, umfassend in einer Menge von mindestens mehr als 10 Gew.-% und weniger als 90 Gew.-% ein penetrationsverstärkendes Agens der nachfolgenden Formel 1

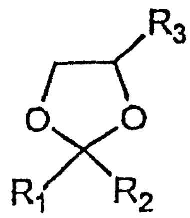

(1)

wobei R1 und R2 identisch oder verschieden sind und ausgewählt sind aus der Gruppe der C1- bis C6-Alkylreste, insbesondere der wahlweise verzweigten, gesättigten C1- bis C4-Alkylreste,
R3 ausgewählt ist aus der Gruppe, bestehend aus Hydroxy-(C1- bis C6-) Alkylresten, insbesondere Hydroxy-(C1- bis C4-) Alkylresten,

- sowie mindestens einen pharmazeutischen Wirkstoff oder dessen pharmazeutisch akzeptables Salz und

- ein weiteres lipophiles penetrationsverstärkendes Agens.

**14.** Zusammensetzung zur transdermalen Verabreichung, umfassend ein penetrationsverstärkendes Agens der nachfolgenden Formel 1

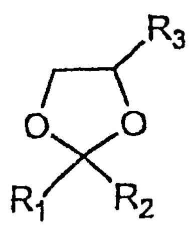

(1)

wobei R1 und R2 identisch oder verschieden sind und ausgewählt sind aus der Gruppe der C1- bis C6-Alkylreste, insbesondere der wahlweise verzweigten, gesättigten C1- bis C4-Alkylreste,
R3 ausgewählt ist aus der Gruppe, bestehend aus Hydroxy-(C1- bis C6-) Alkylresten, insbesondere Hydroxy- bis C4-) Alkylresten,

- sowie mindestens einen pharmazeutischen Wirkstoff oder dessen pharmazeutisch akzeptables Salz und

- ein weiteres lipophiles penetrationsverstärkendes Agens, ausgenommen Ölsäure, dessen Lipophilie, gemessen mittels Bestimmung des Wasseraufnahmevermögens, im Bereich von 0 bis 1,4 Gew.-%, bevorzugt im Bereich von 0,001 bis 0,330 Gew.-% oder im Bereich von 0,340 bis 1,400 Gew.-% liegt.

**15.** Zusammensetzung nach Anspruch 13 oder 14, wobei das penetrationsverstärkende Agens der Formel 1 in einem Anteil von mehr als 10 bis 50 Gew.-%, bevorzugt mehr als 10 bis 25 Gew.-%, am allerbevorzugtesten 15 bis 25 Gew.-% enthalten ist.

**16.** Zusammensetzung nach einem der Ansprüche 13 bis 15, wobei das penetrationsverstärkende Agens der Formel 1 Solketal (2,2-Dimethyl-4-hydroxymethyl-1,3-dioxolan) ist.

**17.** Zusammensetzung nach einem der vorhergehenden Ansprüche 13 bis 16, des weiteren enthaltend einen Haftkleber, bevorzugt einen Haftkleber auf der Basis von Mischungen, enthaltend (Co-)Polymere auf der Basis von Bestandteilen, welche ausgewählt sind aus der Gruppe von C1- bis C6-Alkyl-(meth)acrylaten, C1-bis C5-hydroxyalkyl-(meth)acrylaten, insbesondere enthaltend einen Haftkleber auf der Basis von Mischungen, enthaltend (Co-)Polymere auf der Basis von Bestandteilen, ausgewählt aus der Gruppe, bestehend aus Vinylacetat, 2-Ethylhexylacrylat, 2-Hydroxyethylacrylat, Glycidylmethacrylat, Butylacrylat, Acrylsäure und Methylacrylat.

**18.** Zusammensetzung nach einem der vorherigen Ansprüche 13 bis 17, **dadurch gekennzeichnet, daß** das weitere

lipophile penetrationsverstärkende Agens ausgewählt ist aus der Gruppe der gesättigten Kohlenwasserstoffe mit 10 bis 30 C-Atomen, der wahlweise ungesättigten Fettalkohole mit 10 bis 30 C-Atomen, der gesättigten oder ungesättigten, ein- oder mehrwertigen Fettsäuren mit 8 bis 30 C-Atomen und deren Ester mit wahlweise ungesättigten Fettalkoholen mit 10 bis 30 C-Atomen sowie Triacylglyceriden mit Fettsäureresten mit 8 bis 22 C-Atomen, bevorzugt mit 5 bis 12 C-Atomen.

**19.** Zusammensetzung nach Anspruch 18, **dadurch gekennzeichnet, daß** das weitere lipophile penetrationsverstärkende Agens ausgewählt ist aus der Gruppe, bestehend aus Dioctylcyclohexan, Dodecanol, 2-Octyldodecanol, 2-Hexyldodecanol, Oleylalkohol, Laurinsäure, Ölsäure, Palmitinsäure, Dioctylether, Isopropylmyristat, Hexyllaurat, Cetearyl-isononanoat. Caprinsäure, Caprinsäure-(C1 - bis C20-)alkylester, Ölsäure-(C1- bis C20-)alkylester, insbesondere Decyloleat, Oleyloleat, Docosensäure-(C1- bis C20-)alkylester.

**20.** Zusammensetzung nach Anspruch 19, **dadurch gekennzeichnet, daß** das weitere lipophile penetrationsverstärkende Agens Ethyloleat ist.

**21.** Zusammensetzung nach einem der vorhergehenden Ansprüche 13 bis 20, **dadurch gekennzeichnet, daß** das weitere lipophile penetrationsverstärkende Agens in einer relativen Menge von mindestens 2 Gew-%, bevorzugt zwischen 5 und 15 Gew.-% enthalten ist.

**22.** Zusammensetzung nach einem der vorhergehenden Ansprüche 13 bis 21, **dadurch gekennzeichnet, daß** ein weiteres hydrophiles penetrationsverstärkendes Agens enthalten ist, das bevorzugt eine Wasserlöslichkeit von mindestens 5 Gew.-% aufweist.

**23.** Zusammensetzung nach Anspruch 22, **dadurch gekennzeichnet, daß** das weitere hydrophile penetrationsverstärkende Agens ausgewählt ist aus der Gruppe, bestehend aus Säureamiden, Polyethylenglykolen, Glykolen, Pyrrolidonen, Polymeren von Pyrrolidonderivaten, insbesondere Nicotinsäureamid oder Harnstoff.

**24.** Zusammensetzung nach einem der Ansprüche 22 bis 23, **dadurch gekennzeichnet, daß** das weitere hydrophile penetrationsverstärkende Agens in einer relativen Menge von 1 bis 10 Gew.-%, bevorzugt 2 bis 5 Gew.-% enthalten ist.

**25.** Zusammensetzung nach einem der vorhergehenden Ansprüche 13 bis 24, **dadurch gekennzeichnet, daß** es sich bei dem Wirkstoff um einen lipophilen Wirkstoff, insbesondere ein steroidales Agens handelt.

**26.** Zusammensetzung nach Anspruch 25, **dadurch gekennzeichnet, daß** das steroidale Agens ein Steroidhomon, insbesondere ausgewählt aus der Gruppe, bestehend aus Corticosteroiden, Sexualhormonen, bevorzugt Estrogene, Gestagene, Androgene, ist, insbesondere aus der Gruppe, bestehend aus Testosteron, Estradiol und deren Derivaten, besonders bevorzugt Testosteron, Estradiol, Ethinylestradiol und Norethisteronacetat.

**27.** Zusammensetzung nach einem der vorhergehenden Ansprüche 13 bis 26, **dadurch gekennzeichnet, daß** die Zusammensetzung den Wirkstoff in zumindest gesättigter Lösung, vorzugsweise in übersättigter Lösung enthält.

**28.** Mittel zur transdermalen Verabreichung, umfassend die Zusammensetzung nach einem der vorherigen Ansprüche 13 bis 27.

**29.** Mittel nach Anspruch 28, **dadurch gekennzeichnet, daß** das Mittel eine Emulsion, Salbe, Creme, Lotion oder transdermales therapeutisches System (TTS) ist.

**30.** TTS nach Anspruch 29, mit einer gegebenenfalls abziehbaren Schutzschicht, mindestens einer haftklebenden Matrixschicht, insbesondere einer haftkleberhaltigen Grundierschicht sowie einer haftkleberhaltigen Hautschicht, einer weiteren Zwischenschicht und einer wahlweise wirkstoffund/oder wasserdampfundurchlässigen Rückschicht.

**31.** TTS nach Anspruch 30, **dadurch gekennzeichnet, daß** die Zwischenschicht von der Grundierschicht und der Hautschicht allseitig überragt wird.

**32.** TTS nach einem der Ansprüche 30 oder 31, **dadurch gekennzeichnet, daß** die Zusammensetzung nach einem der Ansprüche 13 bis 27 auf drei Kompartimente aufgeteilt vorliegt, nämlich die haftkleberhaltige Grundierschicht,

die haftkleberhaltige Hautschicht und die weitere Zwischenschicht.

**33.** Verfahren zur Herstellung des TTS nach einem der Ansprüche 30 bis 32 umfassend die Schritte, daß man

- ein erstes Laminat herstellt, indem man eine erste Mischung enthaltend den mindestens einen Wirkstoff und einen wahlweise quervernetzbaren Haftkleber herstellt und auf einem Schutzschicht-Trägermaterial aufbringt,
- ein zweites Laminat herstellt, indem man die erste Mischung auf einem Rückschicht-Trägermaterial aufbringt,
- aus einem weiteren Trägermaterial, bevorzugt einem Vlies, eine Formscheibe ausstanzt und auf dem ersten Laminat aufbringt,
- und eine zweite Mischung, enthaltend das penetrationsverstärkende Agens gemäß Formel 1 und das weitere lipophile penetrationsverstärkende Agens, auf dem weiteren Trägermaterial aufbringt,
- das zweite Laminat darüberlaminiert und Einzel-TTS daraus herstellt.

**34.** Erzeugnis nach einem der Ansprüche 13 bis 32 zur Anwendung in der Substitutionstherapie, insbesondere Hormonsubstitutionstherapie.

**35.** Erzeugnis nach Anspruch 34 zur Anwendung bei Hypogonadismus, Anämie, angeborenem Angiödem, Impotenz, Unfruchtbarkeit oder Empfängnisverhütung.

## Claims

**1.** Transdermal therapeutic system for application to the skin and/or mucosa consisting of at least one active substance in the form of a solid dispersion in combination with at least one destructuring agent, which destructuring agent, if used on its own, would result in a relaxation time of the transdermal therapeutic system of more than 120 ms, and/or at least one structuring agent, which structuring agent, if used on its own, would result in a relaxation time of the transdermal therapeutic system of less than 119 ms, in a common matrix.

**2.** Transdermal therapeutic system according to Claim 1, **characterized in that** the solid dispersion is a molecular dispersion of the active substance in an inert carrier substance.

**3.** Transdermal therapeutic system according to Claim 2, **characterized in that** the inert carrier substance is selected from inert carriers known per se for solid dispersions, such as, for example, sucrose, lactose, succinic acid, polyethylene glycols, polyvinylpyrrolidone, urea, mannitol, mannitose or mixtures thereof.

**4.** Transdermal thereapeutic system according to any of Claims 1 to 3, **characterized in that** the destructuring agent is selected from the group of carboxamides such as urea, nicotinamide, succinamide, methylacetamide, ethylacetamide or mixtures thereof.

**5.** Transdermal therapeutic system according to Claim 4, **characterized in that** the relaxation time is more than 120 ms, preferably more than 150 ms.

**6.** Transdermal therapeutic system according to any of Claims 1 to 5, **characterized in that** the structuring agent is selected from the group of polyols such as, for example, glycerol, ethylene glycol, propylene glycol, from the group of sugar alcohols such as, for example, sorbitol and/or from the group of sugars such as, for example, sucrose or glucose or mixtures thereof.

**7.** Transdermal therapeutic system according to Claim 6, **characterized in that** the relaxation time is less than 119 ms, preferably less than 80 ms.

**8.** Transdermal therapeutic system according to any of the preceding claims, **characterized in that** the ratio between destructuring agent and structuring agent is from 10:1 to 1:10.

**9.** Transdermal therapeutic system according to Claim 8, **characterized in that** the ratio between destructuring agent

and structuring agent is from 2:1 to 1:2.

**10.** Transdermal therapeutic system according to any of the preceding claims, **characterized in that** the active substance is selected from hormones, immunomodulators, immunosuppressants, antibiotics, cytostatics, diuretics, gastrointestinal agents, cardiovascular agents and neuropharmaceuticals or mixtures thereof.

**11.** Transdermal therapeutic system according to Claim 10, **characterized in that** the active substance is selected from hormones such as testosterone, estradiol, estriol, norethisterone, dienogest or mixtures thereof.

**12.** Transdermal therapeutic system according to any of Claims 1 to 11, **characterized in that** the matrix is a sheet-like adhesive piece of material, a plaster, a patch, a gel, an ointment, a cream, an emulsion, an embrocation, a paint or impregnated fabric.

**13.** Composition for transdermal administration comprising in an amount of at least more than 10% by weight and less than 90% by weight a penetration-enhancing agent of the following formula 1

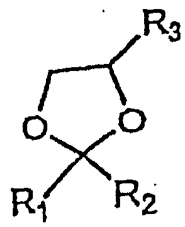

(1)

where $R_1$ and $R_2$ are identical or different and are selected from the group of $C_1$- to $C_6$-alkyl radicals, in particular the optionally branched, saturated $C_1$-to $C_4$-alkyl radicals,
$R_3$ is selected from the group consisting of hydroxy-($C_1$- to $C_6$-)alkyl radicals, in particular hydroxy-($C_1$-to $C_4$-)alkyl radicals,

- and at least one pharmaceutical active substance or its pharmaceutically acceptable salt and
- another lipophilic penetration-enhancing agent.

**14.** Composition for transdermal administration comprising a penetration-enhancing agent of the following formula 1

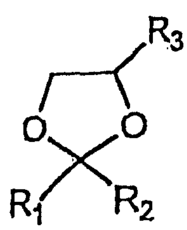

(1)

where $R_1$ and $R_2$ are identical or different and are selected from the group of $C_1$- to $C_6$-alkyl radicals, in particular the optionally branched, saturated $C_1$-to $C_4$-alkyl radicals,
$R_3$ is selected from the group consisting of hydroxy-($C_1$- to $C_6$-)alkyl radicals, in particular hydroxy-($C_1$- to $C_4$-) alkyl radicals,

- and at least one pharmaceutical active substance or its pharmaceutically acceptable salt and
- another lipophilic penetration-enhancing agent, excepting oleic acid, whose lipophilicity measured by determining the water absorption capacity is in the region of 0 to 1.4% by weight, preferably in the region of 0.001 to 0.330% by weight or in the region of 0.340 to 1.400% by weight.

**15.** Composition according to Claim 13 or 14, where the penetration-enhancing agent of the formula 1 is present in a content of more than 10 to 50% by weight, preferably more than 10 to 25% by weight, most preferably 15 to 25% by weight.

**16.** Composition according to any of Claims 13 to 15, where the penetration-enhancing agent of the formula 1 is Solketal (2,2-dimethyl-4-hydroxymethyl-1,3-dioxolane).

**17.** Composition according to any of the preceding Claims 13 to 16, additionally containing a contact adhesive, preferably a contact adhesive based on mixtures containing (co)polymers based on constituents which are selected from the group of $C_1$- to $C_6$-alkyl (meth)acrylates, $C_1$- to $C_5$-hydroxyalkyl (meth)acrylates, in particular containing a contact adhesive based on mixtures containing (co)polymers based on constituents selected from the group consisting of vinyl acetate, 2-ethylhexyl acrylate, 2-hydroxyethyl acrylate, glycidyl methacrylate, butyl acrylate, acrylic acid and methyl acrylate.

**18.** Composition according to any of the preceding Claims 13 to 17, **characterized in that** the other lipophilic penetration-enhancing agent is selected from the group of saturated hydrocarbons with 10 to 30 C atoms, of the optionally unsaturated fatty alcohols with 10 to 30 C atoms, of the saturated or unsaturated, monobasic or polybasic fatty acids with 8 to 30 C atoms and their esters with optionally unsaturated fatty alcohols with 10 to 30 C atoms, and triacylglycerides with fatty acid residues with 8 to 22 C atoms, preferably with 5 to 12 C atoms.

**19.** Composition according to Claim 18, **characterized in that** the other lipophillic penetration-enhancing agent is selected from the group consisting of dioctylcyclohexane, dodecanol, 2-octyldodecanol, 2-hexyldodecanol, oleyl alcohol, lauric acid, oleic acid, palmitic acid, dioctyl ether, isopropyl myristate, hexyl laurate, cetearyl isononanoate, capric acid, ($C_1$- to $C_{20}$-)alkyl caprates, ($C_1$- to $C_{20}$-)alkyl oleates, in particular decyl oleate, oleyl oleate, ($C_1$- to $C_{20}$-)alkyl docosenoates.

**20.** Composition according to Claim 19, **characterized in that** the other lipophilic penetration-enhancing agent is ethyl oleate.

**21.** Composition according to any of the preceding Claims 13 to 20, **characterized in that** the other lipophilic penetration-enhancing agent is present in a relative amount of at least 2% by weight, preferably between 5 and 15% by weight.

**22.** Composition according to any of the preceding Claims 13 to 21, **characterized in that** another hydrophilic penetration-enhancing agent which preferably has a solubility of at least 5% by weight in water is present.

**23.** Composition according to Claim 22, **characterized in that** the other hydrophilic penetration-enhancing agent is selected from the group consisting of amides, polyethylene glycols, glycols, pyrrolidones, polymers of pyrrolidone derivatives, in particular nicotinamide or urea.

**24.** Composition according to any of Claims 22 to 23, **characterized in that** the other hydrophilic penetration-enhancing agent is present in a relative amount of from 1 to 10% by weight, preferably from 2 to 5% by weight.

**25.** Composition according to any of the preceding Claims 13 to 24, **characterized in that** the active substance is a lipophilic active substance, in particular a steroidal agent.

**26.** Composition according to Claim 25, **characterized in that** the steroidal agent is a steroid hormone, in particular selected from the group consisting of corticosteroids, sex hormones, preferably oestrogens, gestagens, androgens, in particular from the group consisting of testosterone, estradiol and its derivatives, particularly preferably testosterone, estradiol, ethinylestradiol and norethisterone acetate.

**27.** Composition according to any of the preceding Claims 13 to 26, **characterized in that** the composition contains the active substance in at least saturated solution, preferably in supersaturated solution.

**28.** Means for transdermal administration comprising the composition according to any of the preceding Claims 13 to 27.

**29.** Means according to Claim 28, **characterized in that** the means is an emulsion, ointment, cream, lotion or transdermal therapeutic system (TTS).

**30.** TTS according to Claim 29, with an optionally detachable protective layer, at least one contact adhesive matrix layer, in particular a contact adhesive-containing primer layer, and a contact adhesive-containing cutaneous layer, another intermediate layer and an optionally active substance- and/or water vapour-impermeable backing layer.

**31.** TTS according to Claim 30, **characterized in that** the primer layer and the cutaneous layer project beyond the intermediate layer on all sides.

**32.** TTS according to either of Claims 30 or 31, **characterized in that** the composition according to any of Claims 13 to 27 is present distributed in three compartments, namely the contact adhesive-containing primer layer, the contact adhesive-containing cutaneous layer and the other intermediate layer.

**33.** Process for producing the TTS according to any of Claims 30 to 32, comprising the steps of

- producing a first laminate by a first mixture containing the at least one active substance and an optionally cross-linkable contact adhesive being produced and applied to a protective layer carrier material,
- producing a second laminate by applying the first mixture to a backing layer carrier material,
- a disc being punched out of another carrier material, preferably a nonwoven, and being applied to the first laminate,
- and a second mixture containing the penetration-enhancing agent according to formula 1 and the other lipophilic penetration-enhancing agent being applied to the other carrier material,
- the second laminate being laminated thereon and single TTS being produced therefrom.

**34.** Product according to any of Claims 13 to 32 for use in replacement therapy, in particular hormone replacement therapy.

**35.** Product according to Claim 34 for use for hypogonadism, anaemia, congenital angioneurotic oedema, impotence, infertility or contraception.

**Revendications**

**1.** Système thérapeutique transdermique pour utilisation sur la peau et/ou la muqueuse composé d'au moins un principe actif sous la forme d'une dispersion solide en combinaison avec au moins une substance déstructurante qui, utilisée toute seule, rendrait le temps de relaxation du système thérapeutique transdermique supérieur à 120 ms, et/ou au moins une substance structurante qui, utilisée toute seule, rendrait le temps de relaxation du système thérapeutique transdermique inférieur à 119 ms, dans une matrice commune.

**2.** Système thérapeutique transdermique selon la revendication 1, **caractérisé en ce que** la dispersion solide est une répartition par dispersion moléculaire du principe actif dans une substance porteuse inerte.

**3.** Système thérapeutique transdermique selon la revendication 2, **caractérisé en ce que** la substance porteuse inerte est choisie parmi des substances porteuses inertes connues en soi pour les dispersions solides, comme le saccharose, le lactose, l'acide succinique, les polyéthylènes glycols, la polyvinylpyrrolidone, l'urée, le mannitol, le mannitose ou des mélanges de ces substances.

**4.** Système thérapeutique transdermique selon l'une quelconque des revendications 1 à 3, **caractérisé en ce que** la substance déstructurante est choisie parmi le groupe des amides de l'acide carboxylique, comme l'urée, l'amide de l'acide nicotinique, l'amide de l'acide succinique, le méthylacétamide, l'éthylacétamide et des mélanges de ces substances.

**5.** Système thérapeutique transdermique selon la revendication 4, **caractérisé en ce que** le temps de relaxation est supérieur à 120 ms, et de préférence supérieur à 150 ms.

**6.** Système thérapeutique transdermique selon l'une quelconque des revendications 1 à 5, **caractérisé en ce que** la substance structurante est choisie parmi le groupe des polyols, par exemple la glycérine, l'éthylène glycol, le propylène glycol, parmi le groupe des alcools de sucres, par exemple le sorbitol et/ou parmi le groupe des sucres, par exemple le saccharose ou le glucose ou des mélanges de ces substances.

**7.** Système thérapeutique transdermique selon la revendication 6, **caractérisé en ce que** le temps de relaxation est inférieur à 119 ms, et de préférence inférieur à 80 ms.

**8.** Système thérapeutique transdermique selon l'une quelconque des revendications précédentes, **caractérisé en ce que** la proportion entre la substance déstructurante et la substance structurante est comprise entre 10 : 1 et 1 : 10.

**9.** Système thérapeutique transdermique selon la revendication 8, **caractérisé en ce que** la proportion entre la substance déstructurante et la substance structurante est comprise entre 2 : 1 et 1 : 2.

**10.** Système thérapeutique transdermique selon l'une quelconque des revendications précédentes, **caractérisé en ce que** le principe actif est choisi parmi les hormones, les immunomodulateurs, les immunosuppresseurs, les antibiotiques, les cytostatiques, les diurétiques, les médicaments gastro-intestinaux, les médicaments cardiovasculaires et les médicaments neurotropes ou des mélanges de ces substances.

**11.** Système thérapeutique transdermique selon la revendication 10, **caractérisé en ce que** le principe actif est choisi parmi les hormones, par exemple la testostérone, l'oestradiol, l'oestriol, la noréthistérone, le dienogest ou des mélanges de ces substances.

**12.** Système thérapeutique transdermique selon l'une quelconque des revendications 1 à 11, **caractérisé en ce que** la matrice est un dispositif adhésif plan, un pansement, un patch, un gel, une pommade, une crème, une émulsion, une friction, un badigeonnage ou un tissu imbibé.

**13.** Composition pour une administration transdermique, comprenant dans une quantité au moins supérieure à 10 % en poids et inférieure à 90 % en poids un agent de renforcement de la pénétration répondant à la formule 1 suivante

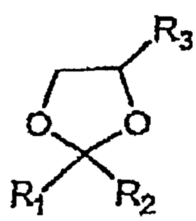

(1)

dans laquelle R1 et R2 sont identiques ou différents et sont choisis dans le groupe des radicaux alkyles en C1 à C6, en particulier les radicaux alkyles en C1 à C4 ramifiés et saturés de manière facultative,

R3 est choisi dans le groupe composé des radicaux hydroxy(alkyl en C1 à C6), en particulier les radicaux hydroxy(alkyl en C1 à C4),

- ainsi qu'au moins un principe actif pharmaceutique ou un sel pharmaceutiquement acceptable de celui-ci et
- un autre agent lipophile de renforcement de la pénétration.

**14.** Composition pour une administration transdermique, comprenant un agent de renforcement de la pénétration répondant à la formule 1 suivante

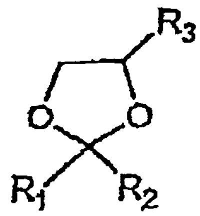

(1)

dans laquelle R1 et R2 sont identiques ou différents et sont choisis dans le groupe des radicaux alkyles en C1 à C6, en particulier les radicaux alkyles en C1 à C4 ramifiés et saturés de manière facultative,

R3 est choisi dans le groupe composé des radicaux hydroxy(alkyl en C1 à C6), en particulier les radicaux hydroxy(alkyl en C1 à C4),

- ainsi qu'au moins un principe actif pharmaceutique ou un sel pharmaceutiquement acceptable de celui-ci et
- un autre agent lipophile de renforcement de la pénétration, à l'exception de l'acide oléique, dont la lipophilie, mesurée par la détermination de la capacité d'absorption de l'eau, est comprise entre 0 et 1,4 % en poids, de préférence dans l'intervalle compris entre 0,001 à 0,330 % en poids ou dans l'intervalle compris entre 0,340 à 1,400 % en poids.

**15.** Composition selon la revendication 13 ou 14, dans laquelle l'agent de renforcement de la pénétration de la formule 1 est contenu dans une proportion de plus de 10 à 50 % en poids, de préférence de plus de 10 à 25 % en poids, et de manière préférée entre toutes entre 15 et 25 % en poids.

**16.** Composition selon l'une quelconque des revendications 13 à 15, dans laquelle l'agent de renforcement de la pénétration de la formule 1 est le solkétal (2,2-diméthyl-4-hydroxyméthyl-1,3-dioxolane).

**17.** Composition selon l'une quelconque des revendications 13 à 16 précédentes, comprenant en outre un adhésif de contact, de préférence un adhésif de contact à base d'un mélange comprenant des (co)polymères à base de composants, lesquels composants sont choisis dans le groupe des (alkyl en C1 à C6)(méth)acrylates, des hydroxy (alkyl en C1 à C5)(méth)acrylates, en particulier comprenant un adhésif de contact à base d'un mélange comprenant des (co)polymères à base de composants choisis dans le groupe composé du vinylacétate, du 2-éthylhexylacrylate, du 2-hydroxyéthylacrylate, du glycidylméthacrylate, du butylacrylate, de l'acide acrylique et du méthylacrylate.

**18.** Composition selon l'une quelconque des revendications 13 à 17 précédentes, **caractérisée en ce que** l'autre agent lipophile de renforcement de la pénétration est choisi dans le groupe des hydrocarbures saturés avec 10 à 30 atomes de carbone, des alcools gras insaturés de manière facultative avec 10 à 30 atomes de carbone, des acides gras saturés ou insaturés, univalents ou polyvalents avec 8 à 30 atomes de carbone, et leurs esters avec des alcools gras insaturés de manière facultative avec 10 à 30 atomes de carbone ainsi que les triacylglycérides avec des radicaux d'acides gras avec 8 à 22 atomes de carbone, de préférence avec 5 à 12 atomes de carbone.

**19.** Composition selon la revendication 18, **caractérisée en ce que** l'autre agent lipophile de renforcement de la pénétration est choisi dans le groupe comprenant le dioctylcyclohexane, le dodécanol, le 2-octyldodécanol, le 2-hexyldodécanol, l'alcool oléique, l'acide laurique, l'acide oléique, l'acide palmitique, le dioctyl éther, le myristate d'isopropyle, le laurate d'hexyle, l'isononanoate de cétéaryle, l'acide caprique, un alkylester (C1 à C20) de l'acide caprique, un alkylester (C1 à C20) de l'acide oléique, en particulier l'oléate de décyle, l'oléate d'oléyle, un alkylester (C1 à C20) de l'acide docosanoïque.

**20.** Composition selon la revendication 19, **caractérisée en ce que** l'autre agent lipophile de renforcement de la pénétration est l'oléate d'éthyle.

**21.** Composition selon l'une quelconque des revendications 13 à 20 précédentes, **caractérisée en ce que** l'autre agent lipophile de renforcement de la pénétration y est contenu en une quantité relative d'au moins 2 % en poids, de préférence entre 5 et 15 % en poids.

**22.** Composition selon l'une quelconque des revendications 13 à 21 précédentes, **caractérisée en ce qu'**elle comprend un autre agent hydrophile de renforcement de la pénétration qui présente de préférence une solubilité dans l'eau d'au moins 5 % en poids.

**23.** Composition selon la revendication 22, **caractérisée en ce que** l'autre agent hydrophile de renforcement de la pénétration est choisi dans le groupe comprenant les amides acides, les polyéthylènes glycols, les glycols, les pyrrolidones, les polymères des dérivés de la pyrrolidone, en particulier l'amide de l'acide nicotinique ou l'urée.

**24.** Composition selon l'une quelconque des revendications 22 à 23, **caractérisée en ce que** l'autre agent hydrophile de renforcement de la pénétration y est contenu en une quantité relative comprise entre 1 et 10 % en poids, de préférence entre 2 et 5 % en poids.

**25.** Composition selon l'une quelconque des revendications 13 à 24 précédentes, **caractérisée en ce que**, concernant le principe actif, il s'agit d'un principe actif lipophile, en particulier d'un agent stéroïdien.

**26.** Composition selon la revendication 25, **caractérisée en ce que** l'agent stéroïdien est une hormone stéroïdienne, en particulier choisie dans le groupe composé des corticostéroïdes, des hormones sexuelles, en particulier des oestrogènes, des progestatifs, les androgènes, en particulier dans le groupe composé de la testostérone, de l'oestradiol et de leurs dérivés, et plus particulièrement de préférence la testostérone, l'oestradiol, l'éthinyloestradiol et l'acétate de noréthistérone.

**27.** Composition selon l'une quelconque des revendications 13 à 26 précédentes, **caractérisée en ce que** la composition contient le principe actif en solution au moins saturée, de préférence dans une solution sursaturée.

**28.** Produit pour une administration transdermique, comprenant la composition selon l'une des revendications 13 à 27 précédentes.

**29.** Produit selon la revendication 28, **caractérisé en ce que** le produit est une émulsion, une pommade, une crème, une lotion ou un système thérapeutique transdermique (TTS).

**30.** Système thérapeutique transdermique selon la revendication 29, muni d'une couche de protection éventuellement détachable, au moins une couche de matrice adhésive, en particulier une couche de base contenant un adhésif de contact ainsi qu'une couche en contact avec la peau contenant un adhésif de contact, une autre couche intermédiaire et une couche dorsale perméable de manière facultative à la vapeur d'eau et/ou à un principe actif.

**31.** Système thérapeutique transdermique selon la revendication 30, **caractérisé en ce que** la couche intermédiaire dépasse la couche de base et la couche en contact avec la peau de tous les côtés.

**32.** Système thérapeutique transdermique selon l'une des revendications 30 ou 31, **caractérisé en ce que** la composition selon l'une quelconque des revendications 13 à 27 se trouve répartie en trois compartiments, c'est-à-dire la couche de base contenant un adhésif de contact, la couche en contact avec la peau contenant un adhésif de contact et l'autre couche intermédiaire.

**33.** Procédé de fabrication du système thérapeutique transdermique selon l'une quelconque des revendications 30 à 32 comprenant les étapes par lesquelles

- un premier stratifié est fabriqué en préparant un premier mélange comprenant le au moins un principe actif et un adhésif de contact de préférence réticulable de manière transversale et en l'appliquant sur une couche de protection composée d'un matériau porteur,
- un deuxième stratifié est fabriqué en appliquant le premier mélange sur un matériau porteur de couche dorsale,
- à partir d'un autre matériau porteur, de préférence un non-tissé, un disque modèle est découpé à l'emporte-pièce et appliqué sur le premier stratifié,
- et un deuxième mélange, comprenant l'agent de renforcement de la pénétration selon la formule I et l'autre agent lipophile de renforcement de la pénétration, est appliqué sur l'autre matériau porteur,
- le deuxième stratifié est laminé par-dessus et des systèmes thérapeutiques transdermiques individuels sont ainsi fabriqués.

**34.** Dispositif selon l'une quelconque des revendications 13 à 32 destiné à être utilisé dans les thérapies de substitution,

en particulier les hormonothérapies substitutives.

**35.** Dispositif selon la revendication 34 destiné à être utilisé en cas d'hypogonadisme, d'anémie, d'oedème angioneurotique héréditaire, d'impuissance, d'infécondité ou de contraception.

*Fig. 1*

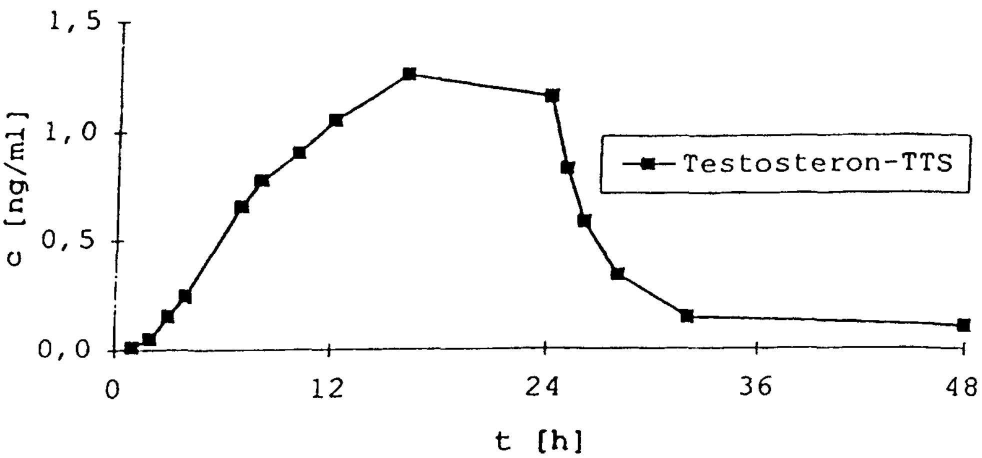
1,5
1,0
0,5
0,0
c [ng/ml]
0
12
24
36
48
t [h]
Testosteron-TTS

*Fig. 2*

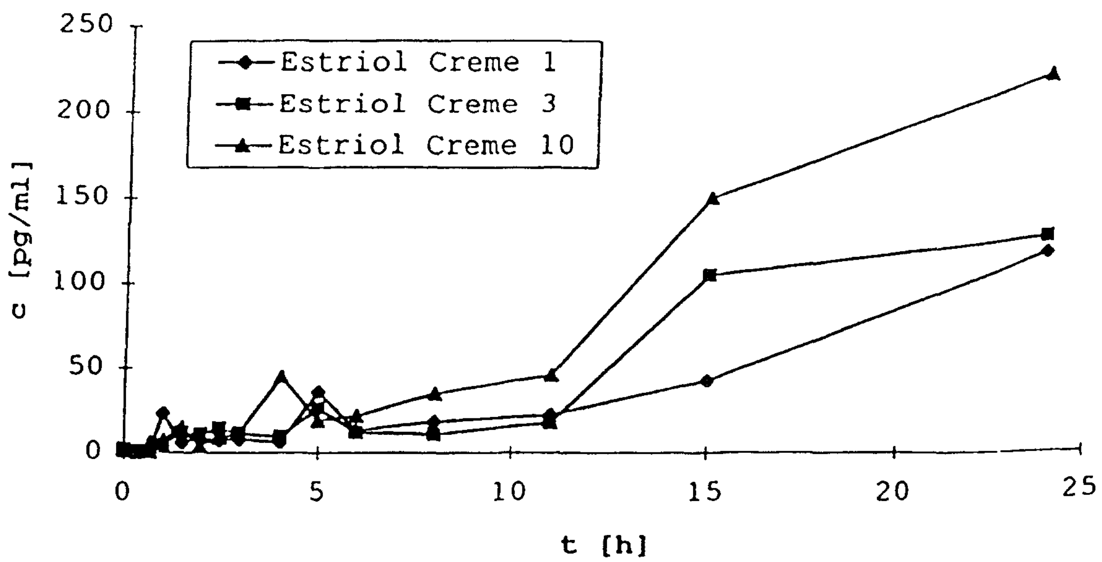
250
200
150
100
50
0
c [pg/ml]
0
5
10
15
20
25
t [h]
Estriol Creme 1
Estriol Creme 3
Estriol Creme 10

*Fig. 3*